(19) Europäisches Patentamt
European Patent Office
Office européen des brevets

(11) **EP 4 190 848 A1**

(12) **EUROPEAN PATENT APPLICATION**
published in accordance with Art. 153(4) EPC

(43) Date of publication:
**07.06.2023 Bulletin 2023/23**

(21) Application number: **21863718.9**

(22) Date of filing: **06.09.2021**

(51) International Patent Classification (IPC):
**C08J 9/42** (2006.01)       **C08J 7/12** (2006.01)
**C08L 5/04** (2006.01)       **C08J 5/18** (2006.01)

(52) Cooperative Patent Classification (CPC):
**A01N 25/10; A01N 33/12; A01N 37/44;
A01N 47/44; A01P 1/00; A01P 3/00;
C08B 37/0084; C08J 5/18; C08J 7/04; C08J 7/06;
C08J 7/12; C08J 9/36; C08J 9/40; C08J 9/42;
C08L 1/02;**                      (Cont.)

(86) International application number:
**PCT/CN2021/116582**

(87) International publication number:
**WO 2022/048656 (10.03.2022 Gazette 2022/10)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **04.09.2020 CN 202010922336**

(71) Applicant: **Oxford University (Suzhou) Science
and Technology CO. LTD
Suzhou, Jiangsu 215123 (CN)**

(72) Inventors:
• **KHAN, Muhammad Kamran
Suzhou, Jiangsu 215123 (CN)**
• **MOLONEY, Mark Gerard
Suzhou, Jiangsu 215123 (CN)**
• **WANG, Dandan
Suzhou, Jiangsu 215123 (CN)**

(74) Representative: **Otten, Roth, Dobler & Partner
mbB Patentanwälte
Großtobeler Straße 39
88276 Berg / Ravensburg (DE)**

(54) **SURFACE-FUNCTIONALIZED MATERIAL AND MODIFIED MATERIAL, AND PREPARATION METHODS THEREFOR AND USE THEREOF**

(57) Provided are an alginate dialdehyde-functionalized material, an alginate dialdehyde-modified material, and preparation methods therefor. Further provided is use of the above materials and methods in the manufacture of antibacterial, antifungal, and antiviral protective products. Raw materials used and the preparation methods are simple, economical, environmentally friendly and easy to scale up, have high inhibitory activity against bacteria, fungi and viruses and have great application potential in the fields of biology, medicine, health, etc.

**(Cont. next page)**

(52) Cooperative Patent Classification (CPC): (Cont.)
    **C08L 5/04; C08L 23/12; C08L 77/06; C09D 5/14;**
    **C09D 105/04**

## Description

### Cross-reference to Related Applications

This application claims the priority to Chinese invention patent application CN 202010922336.9, entitled "Surface-functionalized material and modified material, and preparation methods therefor and use thereof' and filed on September 4, 2020, the entirety of which is incorporated herein by reference.

### Field of the Invention

The present invention relates to the field of materials, in particular to an alginate dialdehyde (ADA for short)-functionalized material and a related modified material, and preparation methods and use thereof.

### Background of the Invention

Pathogenic microorganisms, including various bacteria, fungi, and viruses, can easily spread, for example, through airborne transmission, contact transmission, droplet transmission, etc., thereby posing a huge threat to human health and even causing widespread disease epidemics. In recent years, there has been a variety of viruses with pandemic potential, such as SARS-CoV, H1N1, MERS-CoV, SARS-CoV-2, etc. There have also been a variety of bacteria and fungi that pose a threat to human health because of their easy transmission between people, such bacteria and fungi being, for example, methicillin-resistant Staphylococcus aureus (MRSA), vancomycin-resistant Enterococcus (VRE), Epidermophyton, Aspergillus, Candida, etc.

Interrupting the spread of pathogenic microorganisms is a key way to protect public health. However, conventional protective means (such as medical masks) have only limited effects. For example, medical masks, due to their relatively large filter pore sizes, are not effective against microorganisms with small particle diameters, especially viruses. Protective products that can effectively block viruses (such as EU standard FFP2 or FFP3 masks and US standard N95 masks) are generally equipped with small-pore filters and sometimes also carry electric charges to realize filtration and electrostatic repulsion. These masks however are expensive to make and may make it difficult for wearers to breathe due to their small pore sizes. Furthermore, masks contaminated by pathogenic microorganisms must undergo special treatment, otherwise they will become a source of infection; and meanwhile these masks can be a great burden to the environment.

Most antiviral, antibacterial and antifungal materials are manufactured in complex processes and using expensive chemicals, making it difficult for them to find large-scale application. Some commonly used antimicrobial agents (such as silver or other nanoparticle impregnants) cannot be used for medical purposes due to their own toxicity, and certain organic solvents used in the preparation of functional materials can also bring a burden to the environment. On the other hand, economical and environmentally friendly antimicrobial materials tend to have a relatively low inhibitory activity. Common antimicrobial materials can only inhibit a narrow spectrum of microorganisms. For example, they are effective only against specific types of Gram-positive bacteria, and cannot effectively inhibit a broad spectrum of microorganisms such as Gram-negative bacteria, fungi and viruses, especially microorganisms with increasing resistance.

### Summary of the Invention

In one aspect of the present invention, an alginate dialdehyde (ADA for short)-functionalized material is provided. The ADA-functionalized material includes:

a support layer, which is formed of one or more porous or non-porous materials; and
a link layer, which is formed of ADA, wherein the ADA is linked to the support layer through covalent bonding, electrostatic adsorption, hydrogen bonding, or any combination thereof;
wherein the link layer is capable of combining with a functional material through covalent bonding, electrostatic adsorption, hydrophilic adsorption, hydrophobic adsorption, hydrogen bonding, or any combination thereof.

In yet another aspect of the present invention, a preparation method of the aforementioned ADA-functionalized material is provided. The preparation method includes the following steps:
mixing a porous or non-porous material with an aqueous solution of ADA, and subjecting the resulting mixture to a reaction at a temperature of 10-100°C and a pH value of 1.5-8 for 10 minutes to 24 hours under agitating at a speed of 60-200 rpm; or, directly applying or spraying an aqueous solution of ADA with a pH value of 1.5-8 to a surface of a porous or non-porous material, followed by drying at a temperature of 10-100°C for 10 minutes to 24 hours, to obtain the ADA-functionalized material.

In further another aspect of the present invention, a preparation method of the aforementioned ADA-modified material is provided. The preparation includes the following steps:

adding the aforementioned ADA-functionalized material or the ADA-functionalized material prepared according to the aforementioned method to an aqueous solution of a functional material, and subjecting the resulting mixture to a reaction at a temperature of 10-100°C and a pH value of 1.5- 8 for 10 minutes to 24 hours under agitating at a speed of 60-200 rpm; or, directly applying or spraying an aqueous solution of a functional material with a pH value of 1.5-8 to the ADA-functionalized material, followed by drying at a temperature of 10-100°C for 10 minutes to 24 hours, to obtain the modified material;
wherein the functional material is capable of being linked to the link layer through covalent bonding, electrostatic adsorption, hydrophilic adsorption, hydrophobic adsorption, hydrogen bonding, or any combination thereof.

In yet another aspect of the present invention, a preparation method of the aforementioned ADA-modified material is provided. The preparation method includes the following steps:

mixing an functional material with an aqueous solution of ADA to obtain an aqueous solution of ADA-functional material; and
subjecting the resulting aqueous solution and a porous or non-porous material to a reaction at a temperature of 10-100°C and a pH value of 1.5-8 for 10 minutes to 24 hours under agitating at a speed of 60-200 rpm, or, directly applying the resulting aqueous solution with a pH value of 1.5-8 to a surface of a porous or non-porous material, followed by drying at a temperature of 10-100°C for 10 minutes to 24 hours, to obtain the modified material;
wherein the functional material is capable of being linked to the link layer through covalent bonding, electrostatic adsorption, hydrophilic adsorption, hydrophobic adsorption, hydrogen bonding, or any combination thereof.

In further another aspect of the present invention, provided is use of the ADA-functionalized material, the ADA-modified material, the preparation methods thereof, or the materials prepared according to the preparation methods, as described in the foregoing aspects, in the manufacture of medical protective products and other antimicrobial products.

## Brief Description of the Drawings

Fig. 1 shows attenuated total reflectance-Fourier transform infrared (ATR-FTIR) spectra of polypropylene-based-ADA-modified antimicrobial materials in one embodiment of the present invention.

Fig. 2 shows ATR-FTIR spectra of cellulose-based-ADA-modified antimicrobial materials in one of the embodiments of the present invention.

Fig. 3 shows ATR-FTIR spectra of nylon-based-ADA-modified antimicrobial materials in one of the embodiments of the present invention.

## Detailed Description of the Embodiments

To make the objectives, technical solutions, and advantages of the present invention clearer, the following gives a further detailed description of the present invention with reference to the accompanying drawings and specific embodiments. It should be appreciated that the specific embodiments described herein are only intended to explain the present invention, rather than limiting the protection scope of the present invention.
Unless otherwise defined, all technical and scientific terms as used herein have the same meaning as commonly interpreted by those skilled in the art of the present invention. Terms as used herein in the specification of the present invention are intended only to describe the specific embodiments, rather than limiting the present invention. The term "and/or" as used herein is intended to include any and all combinations of one or more related listed items.
The inventors of the present invention found that alginate dialdehyde (ADA) is a viscous polymer and can be firmly linked to the surface of different materials by any one of at least the following ways: (1) being physically adhered to the surface; (2) forming, through two aldehyde groups in each monomer, a covalent bond with an amino group or an amide group on the surface; (3) being linked, by electrostatic bonding, to the surface with positive charges through negative charges it has due to the presence of hydroxyl and carboxyl groups; and (4) forming a hydrogen bond and linked to the surface.
The inventors of the present invention also found that the surface coated with ADA can combine with a functional material through covalent bonding, electrostatic adsorption, hydrophilic adsorption, hydrophobic adsorption, hydrogen bonding, etc. Therefore, in some embodiments of the present invention, an ADA-functionalized material is provided. The ADA-functionalized material includes a support layer formed of a porous or non-porous material and a link layer formed of ADA.

In some embodiments of the present invention, there is also provided an ADA-modified material including the aforementioned ADA-functionalized material and a functional layer. The functional layer may be a functional material. In some of these embodiments, the functional material is an antimicrobial material.

In some embodiments of the present invention, there are also provided various preparation methods of the aforementioned ADA-functionalized material and ADA-modified material.

Further, the present invention provides use of the above-mentioned materials and preparation methods in the manufacture of antimicrobial products.

The ADA used in the present invention may be obtained from algae, such as brown algae or derivatives thereof, by various methods. In some embodiments, the ADA in the present invention has a molecular weight of about 10,000-150,000 g/mol.

In some embodiments, the ADA is prepared by a method as follows. An appropriate amount of alginic acid or sodium alginate (1-40% w/v) is taken and dissolved in deionized water or ultra-pure (Mill Q) water (200-4000 mL), followed by adding 50-800 mL of ethanol (90-98% v/v) and sodium (m-)periodate ($NaIO_4$, 2-100g). The resulting solution is continuously stirred or agitated and subjected to a reaction at 15-80°C for 12-34 hours under a dark condition. Ethylene glycol (20-100 mL) is added, and the reaction is continued at 15-80°C for 1-5 hours under stirring or agitating to reduce excess periodate. In order to precipitate ADA, 5-20 g of a sodium salt (such as sodium chloride) and 800-2000 mL of 70-98% ethanol are added, and the resulting solution is stirred for 1-2 hours and left to stand for 1-6 hours. After ADA precipitates, ADA is filtered out and washed with 20-60% ethanol 3 times. The ADA solution is dialyzed using a dialysis membrane or a dialysis tube (MWCO 1000-3500 Da) or a desalting column and deionized water or ultra-pure water to remove the sodium salt, thereby obtaining ADA. The obtained ADA can be used directly or stored at -4°C to 25°C for further use, or can be freeze-dried with a freeze dryer to remove water to obtain ADA powder.

In a first aspect of the present invention, there is provided an ADA-functionalized material which includes a support layer and a link layer. The support layer is formed of one or more porous or non-porous materials, and the link layer is formed of ADA. The ADA is linked to the support layer through covalent bonding, electrostatic adsorption, hydrogen bonding, or any combination thereof. The link layer can combine with a functional material through covalent bonding, electrostatic adsorption, hydrophilic adsorption, hydrophobic adsorption, hydrogen bonding, or any combination thereof.

In some embodiments, a mass ratio of the link layer to the support layer is about 1: 100 to 1:10. In some embodiments, the mass ratio of the link layer to the support layer is about 1:50 to 1:20.

In a second aspect of the present invention, there is provided an ADA-modified material which includes the above ADA-functionalized material and a functional layer. The functional layer is formed of a functional material that can be linked to the link layer through covalent bonding, electrostatic adsorption, hydrophilic adsorption, hydrophobic adsorption, hydrogen bonding, or any combination thereof. In some embodiments, the functional material is an antimicrobial material. An ADA-modified functional material or an ADA-modified antimicrobial material is thus obtained.

In some embodiments, the antimicrobial material in the functional layer of the ADA-modified material accounts for 0.01-10% of a total mass of the modified material. In some embodiments, the antimicrobial material in the functional layer accounts for 0.1-5% of the total mass of the modified material.

In some embodiments of the present invention, the porous or non-porous material is one or more selected from the group consisting of polymers, composite materials, and combinations thereof. In some embodiments, the porous or non-porous material may be a polymer, a composite material, a membrane material, a sheet material, a filter material, a non-woven fiber, or any combination thereof. The porous or non-porous material may have a pore size of any size. In some embodiments, the porous or non-porous material may have a pore size of 0.001-10 $\mu$m.

In some embodiments of the present invention, the polymer or the composite material may be selected from the group consisting of polypropylene, cellulose, regenerated cellulose, polyvinylidene fluoride (PVDF), polyether sulfone (PES), polystyrene, polytetrafluoroethylene (PTFE), polyethylene, polyimide, polyamide (such as polyhexamethylene adipamide, commonly known as Nylon 66), and combinations thereof.

In some embodiments of the present disclosure, the functional material may be an antimicrobial material, such as an antimicrobial chemical substance, so that an efficient and long-acting modified antimicrobial material can be formed.

"Antimicrobial" in the present invention means killing or inhibiting microorganisms. The microorganisms may for example be selected from the group consisting of bacteria, fungi, viruses, and combinations thereof, but are not limited thereto. In some embodiments, the bacteria may be Gram-positive bacteria and/or Gram-negative bacteria. In some embodiments, the bacteria are selected from *Klebsiella pneumoniaem, Chlamydia pneumoniae, Streptococcus pneumoniae, Mycobacterium tuberculosis, Streptococcus Group A, Corynebacterium diphtheriae, Hemophilus influenzae, Neisseria meningitidis, Clostridium difficile,* methicillin-resistant *Staphylococcus aureus* (MRSA), vancomycin-resistant *Enterococci* (VRE), *Acinetobacter baumannii,* etc. In some embodiments, the fungi are selected from *Pneumocystis, Aspergillus* (such as *Aspergillus Niger), Coccidioides, Blastomyces, Candida, Mucor, Sporothrix, Epidermophyton,* etc. In some embodiments, the viruses are selected from respiratory syncytial (sin-SISH-uhl) virus, hepatitis virus, varicella virus, polio virus, variola virus, measles virus, mumps virus, chlamydia trachomatis, influenza virus (such as Myxovirus influenzae), SARS-CoV virus, SARS-CoV-2 virus, H1N1 virus, H5N1 virus, H5N7 virus, MERS-CoV virus, Ebola virus, etc.

In some embodiments of the present invention, the antimicrobial material may be selected from the group consisting of amino acids, quaternary ammonium compounds, chlorhexidine compounds, alexidine compounds, biguanide compounds, and combinations thereof.

In some embodiments of the present invention, the amino acids may be selected from the group consisting of cysteine, tyrosine, lysine, arginine, aspartic acid, and combinations thereof. Amino acids with a low pKa give the surface an acidic characteristic. Besides, amino acids are zwitterions and therefore have antimicrobial effects, and it is also possible that amino acids destroy viral envelopes in a similar way.

In some embodiments of the present invention, the quaternary ammonium compounds are selected from the group consisting of alkyl dimethyl benzyl ammonium chloride, alkyl didecyl dimethyl ammonium chloride, dialkyl dimethyl ammonium chloride, dialkyl quaternary ammonium salts, and combinations thereof. In some embodiments, the quaternary ammonium compounds are selected from the group consisting of benzalkonium chloride, cetyl trimethyl ammonium chloride (HTMA-Cl), stearyl dimethyl benzyl ammonium chloride (SMBA-Cl ), didecyl dimethyl ammonium bromide, dioctyl dimethyl ammonium bromide, and combinations thereof. The quaternary ammonium compounds can be used as an antiviral, antibacterial, and antifungal agent in the form of a solution. Quaternary ammonium compounds play antibacterial and antimicrobial effects by inactivating energy-producing enzymes, denaturing essential cell proteins and destroying cell materials. The principle underlying it is to destroy interactions between molecules.

In some embodiments of the present invention, the chlorhexidine compounds may be chlorhexidine digluconate and/or chlorhexidine dihydrochloride.

In some embodiments of the present invention, the alexidine compound is alexidine dihydrochloride.

In some embodiments of the present invention, the biguanide compound is 1-(3-chlorophenyl) biguanide hydrochloride.

Chlorhexidine compounds, alexidine compounds, or biguanide compounds are used as antiviral, antibacterial, and antifungal agents. In the case of a bacterium, as commonly understood, molecules of these compounds bind to the cell wall of the bacterium and destroy the stability of the cell wall. The molecules can affect the integrity of the cell wall at a low concentration. After the cell wall is damaged, the molecules enter the cell itself and attack substances in the cytoplasm (internal substances). Damage to the semitransparent substance of the cytoplasm can lead to leakage of internal substances, thereby causing death of the cell. Similarly, in the case of a fungus, these compounds damage the integrity of the cell wall and substances in the cytoplasm, enter the cytoplasm, and cause leakage of substances inside the cell and thus death of the cell. Likewise, in the case of a virus, these compounds can kill the virus by destroying the structure of the virus.

In some embodiments of the present disclosure, an ADA-modified antibacterial material is provided. The ADA-modified antibacterial material is selected, for example, from the group consisting of polypropylene-based-ADA-cysteine, polypropylene-based-ADA-lysine, polypropylene-based-ADA-benzalkonium chloride, polypropylene-based-ADA-chlorhexidine, polypropylene-based-ADA-phenyl biguanide, cellulose-based-ADA-cysteine, cellulose-based-ADA-lysine, cellulose-based-ADA-benzalkonium chloride, cellulose-based-ADA-chlorhexidine, cellulose-based-ADA-phenyl biguanide, nylon-based-ADA-cysteine, nylon-based-ADA-lysine, nylon-based-ADA-benzalkonium chloride, nylon-based-ADA-chlorhexidine, nylon-based-ADA-phenyl biguanide, and combinations thereof.

In some embodiments of the present disclosure, an ADA-modified antifungal material is provided. The ADA-modified antifungal material is selected, for example, from the group consisting of polypropylene-based-ADA-cysteine, polypropylene-based-ADA-lysine, polypropylene-based-ADA-benzalkonium chloride, polypropylene-based-ADA-chlorhexidine, polypropylene-based-ADA-phenyl biguanide, cellulose-based-ADA-cysteine, cellulose-based-ADA-lysine, cellulose-based-ADA-benzalkonium chloride, cellulose-based-ADA-chlorhexidine, cellulose-based-ADA-phenyl biguanide, nylon-based-ADA-cysteine, nylon-based-ADA-lysine, nylon-based-ADA-benzalkonium chloride, nylon-based-ADA-chlorhexidine, nylon-based-ADA-phenyl biguanide, and combinations thereof.

In some embodiments of the present disclosure, an ADA-modified antiviral material is provided. The ADA-modified antiviral material is selected, for example, from the group consisting of polypropylene-based-ADA-benzalkonium chloride, polypropylene-based-ADA-chlorhexidine, nylon-based-ADA-cysteine, nylon-based-ADA-lysine, nylon-based-ADA-benzalkonium chloride, nylon-based-ADA-chlorhexidine, and combinations thereof.

In some embodiments of the present disclosure, the ADA-modified antimicrobial material of the present disclosure, after being determined of its antibacterial activity based on "ISO 20743:2013 Textiles-Determination of antibacterial activity of textile products", is found to have achieved at least a 3-log or more reduction of a bacterium. In some embodiments, the material is determined based on ISO 20743:2013 to have achieved a (3.0-4.5)-log reduction of a bacterium. In some embodiments, a (4.0-4.4)-log reduction of a bacterium is achieved. In some embodiments, the ADA-modified antimicrobial material is a polypropylene-based-ADA-modified antimicrobial material or a cellulose-based-ADA-modified antimicrobial material. In some embodiments, the ADA-modified antimicrobial material is selected from polypropylene-based-ADA-benzalkonium chloride, polypropylene-based-ADA-chlorhexidine, cellulose-based-ADA-benzalkonium chloride, and cellulose-based-ADA-chlorhexidine. In some embodiments, the Gram-positive bacteria are *Staphylococcus aureus.* In some embodiments, the Gram-negative bacteria are *Klebsiella pneumoniae.*

In some embodiments of the present disclosure, the ADA-modified antimicrobial material of the present disclosure, after being determined of its antiviral activity, is found to have achieved at least a 4-log or more reduction of a virus. In some embodiments, a (4-6)-log reduction of a virus is achieved. In some embodiments, a (4.2-5.9)-log reduction of a virus is achieved. In some embodiments, the ADA-modified antimicrobial material is a polypropylene-based-ADA-modified antimicrobial material or a nylon ADA-modified antimicrobial material. In some embodiments, the ADA-modified antimicrobial material is selected from polypropylene-based-ADA-benzalkonium chloride and nylon-based-ADA-benzalkonium chloride. In some embodiments, the virus is novel coronavirus SARS-CoV-2.

In some embodiments of the present disclosure, the ADA-modified antimicrobial material of the present disclosure, after being determined of its antiviral activity based on "ISO 18184:2019 Textiles-Determination of antiviral activity of textile products", is found to have achieved at least a 3-log or more reduction of a virus. In some embodiments, the material is determined based on ISO 18184:2019 to have achieved a (3.0-6.0)-log reduction of a virus. In some embodiments, a (3.1-5.7)-log reduction of a virus is achieved. In some embodiments, a (5.3-5.7)-log reduction of a virus is achieved. In some embodiments, the ADA-modified antimicrobial material is a polypropylene-based-ADA-modified antimicrobial material or a cellulose-based-ADA-modified antimicrobial material. In some embodiments, the ADA-modified antimicrobial material is a polypropylene-based-ADA-modified antimicrobial material or a cellulose ADA-modified antimicrobial material. In some embodiments, the ADA-modified antimicrobial material is selected from polypropylene-based-ADA-benzalkonium chloride, polypropylene-based-ADA-chlorhexidine, cellulose-based-ADA-benzalkonium chloride, and cellulose-based-ADA-chlorhexidine. In some embodiments, the virus is H1N1 influenza virus.

In a third aspect of the present invention, there is provided a method for preparing the above-described ADA-functionalized material. The method includes the following steps: mixing a porous or non-porous material with an aqueous solution of ADA, and then subjecting the resulting mixture to a reaction at a temperature of 10-100°C and a pH value of 1.5-8 for 10 minutes to 24 hours under agitating at a speed of 120-200 rpm.

In some embodiments, a mass ratio of the ADA contained in the aqueous solution of ADA to the porous or non-porous material is 1: 100 to 1:10. In some embodiments, the mass ratio is 1:50 to 1:20.

In some embodiments, the aqueous solution of ADA has a pH value of 3-6. In some embodiments, the temperature is 40-80°C. In some embodiments, the agitating is conducted at a speed of 120-200 rpm. In some embodiments, a time period for the reaction is 8-16 hours.

In some embodiments, the aqueous solution of ADA has a concentration of 100-1000 mg/ml and is adjusted to have an appropriate pH value with an acid (such as sulfuric acid, hydrochloric acid, acetic acid, phosphoric acid, nitric acid). In some embodiments, the concentration is 50-500 mg/ml. In some embodiments, the concentration is 20-100 mg/ml.

In the third aspect of the present invention, alternatively, the method includes the following steps: directly applying or spraying an aqueous solution of ADA with a pH value of 1.5-8 to a surface of a porous or non-porous material, followed by drying at a temperature of 10-100°C for 10 minutes to 24 hours to obtain the ADA-functionalized material.

In some embodiments, a mass ratio of the ADA contained in the aqueous solution of ADA to the porous or non-porous material is 1: 100 to 1:10. In some embodiments, the mass ratio is 1:50 to 1:20.

In some embodiments, the temperature is 10-40°C. In some embodiments, a time period for the drying is 10 minutes to 1 hour. In some embodiments, the aqueous solution of ADA has a pH value of 3-6.

In some embodiments, the aqueous solution of ADA has a concentration of 10-1000 mg/ml and is adjusted to have an appropriate pH value with an acid (such as sulfuric acid, hydrochloric acid, acetic acid, phosphoric acid, nitric acid). In some embodiments, the concentration is 50-500 mg/ml.

In a fourth aspect of the present invention, there is provided a method for preparing the ADA-modified material described above. The method includes the following steps: adding the aforementioned ADA-functionalized material or the ADA-functionalized material prepared according to the aforementioned method to an aqueous solution of a functional material, and subjecting the resulting mixture to a reaction at a temperature of 10-100°C and a pH value of 1.5-8 for 10 minutes to 24 hours under agitating at a speed of 60-200 rpm to obtain the modified material. The functional material is capable of being linked to the link layer through covalent bonding, electrostatic adsorption, hydrophilic adsorption, hydrophobic adsorption, hydrogen bonding, or any combination thereof.

In some embodiments, a mass ratio of the functional material contained in the aqueous solution of the functional material to the ADA-functionalized material is 1: 100 to 1:10. In some embodiments, the mass ratio is 1:50 to 1:20.

In some embodiments, the aqueous solution of the functional material has a pH value of 3-6. In some embodiments, the temperature is 40-60°C. In some embodiments, the agitating is conducted at a speed of 120-200 rpm. In some embodiments, a time period for the reaction is 8-16 hours.

In some embodiments, the aqueous solution of the functional material has a concentration of 10-100 mg/ml and is adjusted to have an appropriate pH value with an acid (such as sulfuric acid, hydrochloric acid, acetic acid, phosphoric acid, nitric acid). In some embodiments, the concentration is 5-60 mg/ml. In some embodiments, the concentration is 2-20 mg/ml.

In some embodiments, the functional material is an antimicrobial material.

In the fourth aspect of the present invention, alternatively, the method includes the following steps: directly applying or spraying an aqueous solution of a functional material to the ADA-functionalized material, followed by drying at a temperature of 10-100°C for 10 minutes to 24 hours to obtain the modified material. The functional material can be linked to the link layer through covalent bonding, electrostatic adsorption, hydrophilic adsorption, hydrophobic adsorption, hydrogen bonding, or any combination thereof. The aqueous solution of the functional material has a pH value of 1.5-8. In some embodiments, a mass ratio of the functional material contained in the aqueous solution of the functional material to the ADA-functionalized material is 1:1000 to 1:10. In some embodiments, the mass ratio is 1:100 to 1:10. In some embodiments, the mass ratio is 1:50 to 1:20.

In some embodiments, the aqueous solution of the functional material has a pH value of 3-6. In some embodiments, the temperature is 30-50°C. In some embodiments, a time period for the drying is 10 minutes to 1 hour.

In some embodiments, the aqueous solution of the functional material has a concentration of 10-100 mg/ml and is adjusted to have an appropriate pH value with an acid (such as sulfuric acid, hydrochloric acid, acetic acid, phosphoric acid, nitric acid). In some embodiments, the concentration is 5-60 mg/ml. In some embodiments, the concentration is 2-20 mg/ml.

In some embodiments, the functional material is an antimicrobial material.

In a fifth aspect of the present invention, there is provided a method for preparing the ADA-modified material described above. The method includes the following steps: mixing a functional material with an aqueous solution of ADA to obtain an aqueous solution of ADA-functional material; subjecting the resulting aqueous solution and a porous or non-porous material to a reaction at a temperature of 10-100°C and a pH value of 1.5-8 for 10 minutes to 24 hours under agitating at a speed of 60-200 rpm to obtain the modified material. The functional material can be linked to the link layer through covalent bonding, electrostatic adsorption, hydrophilic adsorption, hydrophobic adsorption, hydrogen bonding, or any combination thereof. In some embodiments, a mass ratio of the functional material to the ADA to the porous or non-porous material is (1-10): (10-100): 1000.

In some embodiments, the aqueous solution of ADA has a pH value of 3-6. In some embodiments, the temperature is 40-70°C. In some embodiments, the agitating is conducted at a speed of 120-200 rpm. In some embodiments, a time period for the reaction is 8-16 hours.

In some embodiments, the aqueous solution of ADA has a concentration of 100-1000 mg/ml and is adjusted to have an appropriate pH value with an acid (such as sulfuric acid, hydrochloric acid, acetic acid, phosphoric acid, nitric acid). In some embodiments, the concentration is 50-500 mg/ml. In some embodiments, the concentration is 20-100 mg/ml.

In some embodiments, the functional material in the aqueous solution of ADA-functional material has a final concentration of 10-100 mg/ml. In some embodiments, the final concentration is 5-60 mg/ml. In some embodiments, the final concentration is 2-20 mg/ml.

In some embodiments, the functional material is an antimicrobial material.

In the method according to the fifth aspect of the present invention, alternatively, the method includes the following steps: mixing a functional material with an aqueous solution of ADA to obtain an aqueous solution of ADA-functional material; directly applying or spraying the resulting aqueous solution with a pH value of 1.5-8 to a surface of a porous or non-porous material, followed by drying at a temperature of 10-100°C for 10 minutes to 24 hours to obtain the modified material. The functional material can be linked to the link layer through covalent bonding, electrostatic adsorption, hydrophilic adsorption, hydrophobic adsorption, hydrogen bonding, or any combination thereof. In some embodiments, a mass ratio of the functional material to the ADA to the porous or non-porous material is (1-10): (10-100): 1000.

In some embodiments, the aqueous solution of ADA has a pH value of 3-6. In some embodiments, the temperature is 30-60°C. In some embodiments, a time period for the drying is 10 minutes to 1 hour.

In some embodiments, the aqueous solution of ADA has a concentration of 100-1000 mg/ml and is adjusted to have an appropriate pH value with an acid (such as sulfuric acid, hydrochloric acid, acetic acid, phosphoric acid, nitric acid). In some embodiments, the concentration is 50-500 mg/ml. In some embodiments, the concentration is 20-100 mg/ml.

In some embodiments, the functional material in the aqueous solution of ADA-functional material has a final concentration of 10-100 mg/ml. In some embodiments, the final concentration is 5-50 mg/ml. In some embodiments, the final concentration is 2-10 mg/ml.

In some embodiments, the functional material is an antimicrobial material.

In some embodiments, each of the methods described in the third, fourth, and fifth aspects of the present invention further includes the steps of: washing the resulting product with water, storing the product in a wet state or drying the product in the air or in an oven.

In a sixth aspect of the present invention, there is provided use of the ADA-functionalized material, the ADA-modified material, or preparation methods thereof described in the foregoing aspects in the manufacture of medical protective products and antimicrobial products.

In some embodiments, the use includes the manufacture of masks, isolation gowns, gloves, personal protective articles, bandages, medical tapes, medical caps, medical bed sheets, medical clothes, air filters, water filters, electronic products, household appliances, vehicle parts, etc.

The ADA-functionalized material, the ADA-modified material, and the preparation methods thereof provided in the present invention have various beneficial effects. For example, the raw materials used are economical, environmentally friendly, and safe; the support layer (the base layer) can come from a variety of common base materials of any pore sizes; the ADA can be derived from algae such as brown algae; and the antimicrobial material can be amino acids, quaternary ammonium salts, chlorhexidine, biguanide compounds and so on which are economical and safe. Further, the preparation methods are simple; the raw materials are easily available and are of low cost, requiring no irritating organic solvents, making the preparation environmentally friendly and easy to scale up. Furthermore, the materials have high inhibitory activities against bacteria (including Gram-positive and negative bacteria), fungi, and viruses, which can reduce the risk of secondary infection. The present invention therefore has great application potential in the fields of biology, medicine, and health.

Chemicals used in the following embodiments, unless otherwise specified, are all commercially available and will not be detailed again.

**Example 1: Preparation of ADA-modified antimicrobial materials**

This example is a preparation example of ADA-modified antimicrobial materials. In this example, a polypropylene-based material, a nylon-based material (taking polyhexamethylene adipamide-based nylon 66 composite material as an example), and a cellulose-based material were taken as examples of the porous or non-porous material, and amino acids (cysteine, lysine), quaternary ammonium compounds (benzalkonium chloride), chlorhexidine, and biguanide compounds (1-(3-chlorophenyl) biguanide hydrochloride) were taken as examples of the antimicrobial material. The polypropylene-based material (product number: MFPP047020) was purchased from Tansole China. The cellulose-based material (product number: GB/T1914-2007) was purchased from General Electronics (GE) Whatman. The nylon-based material (polyhexamethylene adipamide, namely Nylon 66) (product number: GNWP04700) was purchased from Merck Millipore.

1. Preparation of ADA

2-10 g of sodium alginate was measured and dissolved in 20-800 mL of deionized water, followed by adding 50-200 mL of ethanol and 2-10 g of sodium periodate. After the resulting solution was subjected to a reaction at 25°C for 24 hours under stirring (200 rpm) and under a dark condition, 5-50 mL of ethylene glycol was added, and the solution was continuously stirred for 2 hours to reduce excess periodate. 5-20 g of sodium chloride and 800 mL of ethanol were then added to precipitate ADA, and the solution was stirred for 30 minutes and left to stand for 1.5 hours. The resulting precipitate was then filtered out and washed with 50% ethanol 3 times. The resulting solution was dialyzed using a dialysis tube (MWCO, 3500Da) and distilled water, and the resulting dialysate was freeze-dried to obtain a final product.

2. Preparation of ADA-functionalized materials

The ADA-functionalized materials each were prepared by one of the following methods.

Method I: 10 g of the above porous or non-porous material was measured, cleaned, and then immersed in an aqueous solution of ADA containing 0.1-1 g of ADA and having a pH value of 3-6. The resulting mixture was then subjected to a reaction at 60°C for 12 hours under agitating at 120 rpm to obtain an ADA-functionalized material. After the reaction, the obtained ADA-functionalized material was washed with distilled water.

Method II: 10 g of the above porous or non-porous material was measured and cleaned. An aqueous solution of ADA containing 0.1 -1 g of ADA and having a pH value of 3-6 was directly applied or sprayed at 60°C to a surface of the material, and then dried at 30°C for 8 hours to obtain an ADA-functionalized material.

The products prepared as above are polypropylene-based-ADA-functionalized materials, cellulose-based-ADA-functionalized materials, and nylon-based-ADA-functionalized materials.

3. Preparation of ADA-modified antimicrobial materials

The ADA-modified antimicrobial materials each were prepared by one of the following methods.

Method I: 10 g of an ADA-functionalized material and 10-1000 mg of an aqueous solution of the aforementioned antimicrobial material with a pH value of 3-6 were measured and subjected to a reaction at 40-60°C for 12 hours under agitating at 120 rpm to obtain an ADA-modified material.

Method II: 10-1000 mg of an aqueous solution of the aforementioned antimicrobial material with a pH value of 3-6 was directly applied or sprayed to a surface of 10 g of an ADA-functionalized material, followed by drying at 40°C for 8 hours to obtain an ADA-modified material.

Method III: 10-100 mg of the antimicrobial material and 0.1-1 g of ADA were measured and mixed, and then mixed with Milli Q ultra-pure water, followed by adjusting the resulting solution to have a pH value of 3-6 to obtain an aqueous solution of ADA-antimicrobial material. 10 g of the porous or non-porous material was measured and was subjected to a reaction with the obtained aqueous solution of ADA-antimicrobial material at 40-70°C for 8-16 hours under agitating at 120-200 rpm to obtain an ADA-modified antimicrobial material.

Method IV: 10-100 mg of the antimicrobial material and 0.1-1 g of ADA were measured and mixed, and then mixed with Milli Q ultra-pure water, followed by adjusting the resulting solution to have a pH value of 3-6 to obtain an aqueous solution of ADA-antimicrobial material. The resulting aqueous solution was directly applied or sprayed to a surface of 10 g of the porous or non-porous material and dried at 40°C for 8 hours to obtain an ADA-modified material.

The products prepared as above are: a modified material polypropylene-based-ADA-cysteine, a modified material polypropylene-based-ADA-lysine, a modified material polypropylene-based-ADA-benzalkonium chloride, a modified material polypropylene-based-ADA-chlorhexidine, a modified material polypropylene-based-ADA-phenyl biguanide; a modified material cellulose-based-ADA-cysteine, a modified material cellulose-based-ADA-lysine, a modified material cellulose-based-ADA-benzalkonium chloride, a modified material cellulose-based-ADA-chlorhexidine, a modified material cellulose-based-ADA-phenyl biguanide; a modified material nylon-based-ADA-cysteine, a modified material nylon-based-ADA-lysine, a modified material nylon-based-ADA-benzalkonium chloride, a modified material nylon-based-ADA-chlorhexidine, and a modified material nylon-based-ADA-phenyl biguanide.

The ADA-modified material products obtained as above were washed several times and then stored at 4°C in distilled water for later use.

**Example 2: ATR-FTIR characterization of the ADA-modified antimicrobial materials**

The ADA-modified antimicrobial materials prepared in Example 1 were tested for their surface chemical structures by the Attenuated Total Reflectance-Fourier Transform Infrared (ATR-FTIR, NEXUS 670, Thermo Nicolet, USA) spectroscopic method. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

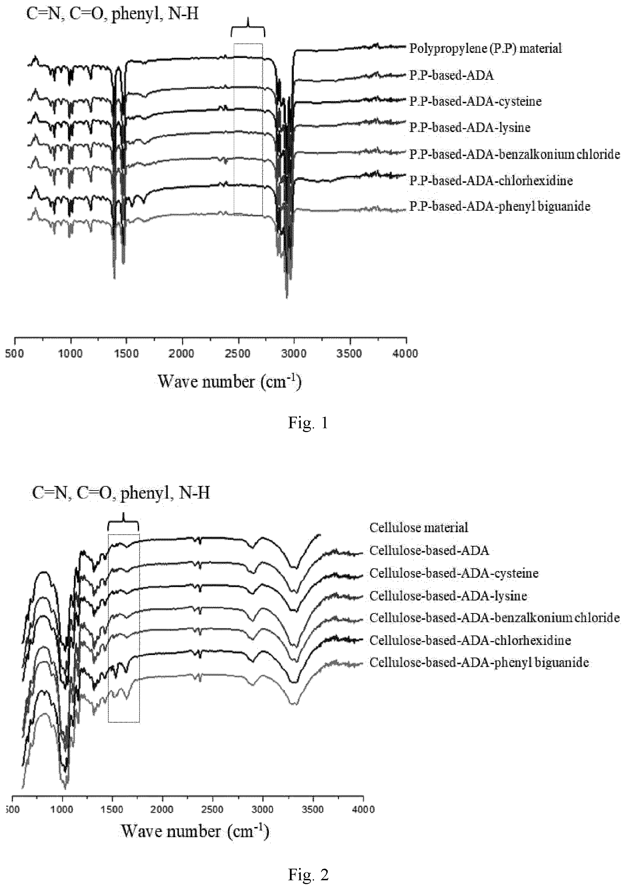

Test results are shown in Figs. 1-3, which indicate successful combinations of the antimicrobial material with the ADA-functionalized materials.

Fig. 1 shows ATR-FTIR spectra of the polypropylene-based-ADA-modified antimicrobial materials. The broad peak at 1610-1720 cm$^{-1}$ represents the C=O of the aldehyde and carboxyl groups on polypropylene-based-ADA. The broad peaks at 1605-1730 cm$^{-1}$ correspond to the formation of C=N (Schiff base) bonds in cysteine and lysine, the C-N bond in benzalkonium chloride, and the C=N bonds in the structures of chlorhexidine and 1-(3-chlorophenyl) biguanide hydrochloride. The peaks at 1512-1570 cm$^{-1}$ correspond to the phenyl groups in benzalkonium chloride, chlorhexidine, and 1-(3-chlorophenyl) biguanide hydrochloride.

Fig. 2 shows ATR-FTIR spectra of the cellulose-based-ADA-modified antimicrobial materials. The broad peak at 1580-1700 cm$^{-1}$ represents the C=O of the aldehyde and carboxyl groups on cellulose-based-ADA, and a similar peak on the cellulose corresponds to OH. The sharp peaks at 1580-1700 cm$^{-1}$ correspond to the formation of C=N Schiff bonds in cysteine and lysine, and the C-N bond in benzalkonium chloride. The strong peaks at 1585-1690 cm$^{-1}$ represent the C=N bonds in the structures of chlorhexidine and 1-(3-chlorophenyl) biguanide hydrochloride. The peaks at 1508-1560 cm$^{-1}$ corresponds to the phenyl groups in benzalkonium chloride, chlorhexidine, and 1-(3-chlorophenyl) biguanide hydrochloride.

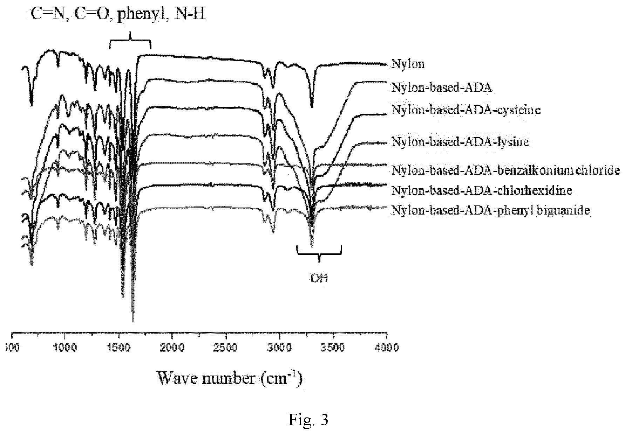

Fig. 3 shows ATR-FTIR spectra of the nylon-based-ADA-modified antimicrobial materials. The two broad peaks at 1500-1580 cm$^{-1}$ and 1580-1700 cm$^{-1}$ correspond to the amide and carboxyl groups in the structures of the nylon composite materials. Since the peaks of the Schiff base C=N, the aldehyde group, and the phenyl group appear in similar regions, which makes it difficult to distinguish the nylon composite materials from the original nylon material. However, the sharp peaks of OH at 3200-3550 cm$^{-1}$ show hydroxyl groups added in nylon-based-ADA, nylon-based-ADA-cysteine, and nylon-based-ADA-lysine.

**Example 3a: Determination of antibacterial activity of the ADA-modified antimicrobial materials**

In this example, the polypropylene-based-ADA-modified antimicrobial materials, the cellulose-based-ADA-modified antimicrobial materials, and the nylon-based-ADA-modified antimicrobial materials prepared in Example 1 were tested for their antibacterial activity. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

I. Test methods

Gram-positive bacterium *Micrococcus lysodeikticus* and Gram-negative bacterium *Escherichia coli (E. coli)* were taken as example bacteria. The polypropylene-based-ADA-modified antimicrobial materials, the cellulose-based-ADA-modified antimicrobial materials, and the nylon-based-ADA-modified antimicrobial materials were tested for their antibacterial activity. Each of the samples was provided in triplicate, and an average value was taken as a result.
*Micrococcus lysodeikticus* and *E. coli* were incubated in an LB (Luria-Bertani) medium overnight at 37°C under stirring. The resulting fresh bacterial culture was diluted into a bacterial suspension with a number of colonies of $10^{-4}$ CFU/ml. 0.1 g of a polymer-ADA-modified antimicrobial material and 10 mL of the bacterial suspension were mixed and placed in a flask for incubation at 37°C for 2 hours in a shaking bed at a shaking speed of 120 rpm. Another 10 mL of the bacterial suspension without the modified material was separately placed in another flask as a control. After the incubation, 10-100 $\mu$l of the bacterial suspension was seeded onto an LB agar plate and incubated at 37°C for 18-36 hours. After 18 hours of incubation, the number of viable colonies was calculated using the plate count method.
The antibacterial activity was calculated based on the following formula:

$$I = \frac{N_0 - N_i}{N_0} \times 100\%$$

where *I* represents the antibacterial activity, $N_0$ represents the number of colonies after the incubation in the control group, and $N_i$ represents the number of colonies after incubation in the treatment group.
In addition, in order to check the stability of the antibacterial coating, all the polymer-ADA-modified antimicrobial materials were immersed in autoclaved Milli Q ultra-pure water for 48 hours, and their antibacterial activity was compared with that of the freshly prepared materials.

II. Test results

1. Polypropylene-based-ADA-modified antimicrobial materials

Results for the antibacterial activity of the polypropylene-based-ADA-modified antimicrobial material against *Micrococcus lysodeikticus* and *E. coli* are shown in Table 1. The polypropylene material alone has an extremely low antibacterial activity. The freshly prepared sample and the 48-hour sample of the polypropylene-based-ADA material exhibit respectively 30% and 31% inhibition against the Gram-positive bacterium and both 25% inhibition against the Gram-negative bacterium. The freshly prepared sample and the 48-hour sample of the polypropylene-based-ADA-cysteine material exhibit respectively 97% and 96% inhibition against the Gram-positive bacterium and 95% and 94% inhibition against the Gram-negative bacterium. The freshly prepared sample and the 48-hour sample of the polypropylene-based-ADA-lysine material exhibit respectively 94% and 92 % inhibition against the Gram-positive bacterium and 87% and 85% inhibition against the Gram-negative bacterium. The freshly prepared samples and the 48-hour samples of the polypropylene-based-ADA-benzalkonium chloride material, the polypropylene-based-ADA-chlorhexidine material, and the polypropylene-based-ADA-phenyl biguanide (1-(3-chlorophenyl) biguanide hydrochloride) almost all exhibit 100% inhibition against both the Gram-positive bacterium and Gram-negative bacterium. It can also be seen that all the polypropylene-based-ADA-modified antimicrobial materials of the present invention, after having been immersed in water for 48 hours, maintain an antibacterial activity almost the same as that of the freshly prepared materials.

Table 1 Test results for antibacterial activity of the polypropylene-based-ADA-modified antimicrobial materials

| Samples | Antibacterial activity of freshly prepared samples against the Gram+ bacterium (%) | Antibacterial activity of 48- hour samples against the Gram+ bacterium (%) | Antibacterial activity of freshly prepared samples against the Gram- bacterium (%) | Antibacterial activity of 48-hour samples against the Gram- bacterium (%) |
|---|---|---|---|---|
| Polypropylene | 9 | 8 | 3 | 3 |
| Polypropylene-based-ADA | 30 | 31 | 25 | 25 |
| Polypropylene-based-ADA-cysteine | 97 | 96 | 95 | 94 |
| Polypropylene-based-ADA-lysine | 94 | 92 | 87 | 85 |
| Polypropylene-based-ADA-benzalkonium chloride | 100 | 100 | 100 | 100 |
| Polypropylene-based-ADA-chlorhexidine | 100 | 100 | 100 | 100 |
| Polypropylene-based-ADA-phenyl biguanide | 100 | 100 | 100 | 99 |

2. Cellulose-based-ADA-modified antimicrobial materials

Results for the antibacterial activity of the cellulose-based-ADA-modified antimicrobial materials against *Micrococcus lysodeikticus* and *E. coli* are shown in Table 2. The cellulose material alone has an extremely low antibacterial activity. The freshly prepared sample and the 48-hour sample of the cellulose-based-ADA material exhibit respectively 35% and 33% inhibition against the Gram-positive bacterium and 15% and 13% inhibition against the Gram-negative bacterium. The freshly prepared sample and the 48-hour sample of the cellulose-based-ADA-cysteine material both exhibit 94% inhibition against the Gram-positive bacterium and 90% inhibition against the Gram-negative bacterium. The freshly prepared sample and the 48-hour sample of the cellulose-based-ADA-lysine material exhibit respectively 91% and 89% inhibition against the Gram-positive bacterium and 85% and 83% inhibition against the Gram-negative bacterium. The freshly prepared samples and the 48-hour samples of the cellulose-based-ADA-benzalkonium chloride material, the cellulose-based-ADA-chlorhexidine material, and the cellulose-based-ADA-phenyl biguanide (1-(3-chlorophenyl) biguanide hydrochloride) almost all exhibit 100% inhibition against both the Gram-positive bacterium and the Gram-negative bacterium. It can also be seen that all the cellulose-based-ADA-modified antimicrobial materials of the present invention, after having been immersed in water for 48 hours, maintain an antibacterial activity almost the same as that of the freshly prepared materials.

Table 2: Test results for antibacterial activity of the cellulose-based-ADA-modified antimicrobial materials

| Samples | Antibacterial activity of freshly prepared samples against the Gram+ bacterium (%) | Antibacterial activity of 48- hour samples against the Gram+ bacterium (%) | Antibacterial activity of freshly prepared samples against the Gram-bacterium (%) | Antibacterial activity of 48-hour samples against the Gram- bacterium (%) |
|---|---|---|---|---|
| Cellulose | 3 | 4 | 3 | 3 |
| Cellulose-based-ADA | 35 | 33 | 15 | 13 |
| Cellulose-based-ADA-cysteine | 94 | 94 | 90 | 90 |

(continued)

| Samples | Antibacterial activity of freshly prepared samples against the Gram+ bacterium (%) | Antibacterial activity of 48- hour samples against the Gram+ bacterium (%) | Antibacterial activity of freshly prepared samples against the Gram-bacterium (%) | Antibacterial activity of 48-hour samples against the Gram- bacterium (%) |
|---|---|---|---|---|
| Cellulose-based-ADA-lysine | 91 | 89 | 85 | 83 |
| Cellulose-based-ADA-benzalkonium chloride | 100 | 100 | 100 | 100 |
| Cellulose-based-ADA-chlorhexidine | 100 | 100 | 100 | 100 |
| Cellulose-based-ADA-phenyl biguanide | 100 | 100 | 98 | 98 |

3. Nylon-based-ADA-modified antimicrobial materials

Results for the antibacterial activity of the nylon-based-ADA-modified antimicrobial material against *Micrococcus lysodeikticus* and E. *coli* are shown in Table 3. The nylon material alone has an extremely low antibacterial activity. The freshly prepared sample and the 48-hour sample of the nylon-based-ADA material exhibit respectively 37% and 36% inhibition against the Gram-positive bacterium and 20% and 18% inhibition against the Gram-negative bacterium. The freshly prepared sample and the 48-hour sample of the nylon-based-ADA-cysteine material both exhibit 95% inhibition against the Gram-positive bacterium and respectively 95% and 94% inhibition against the Gram-negative bacterium. The freshly prepared sample and the 48-hour sample of the nylon-based-ADA-lysine material exhibit respectively 91% and 90 % inhibition against the Gram-positive bacterium and 88% and 87% inhibition against the Gram-negative bacterium. The freshly prepared samples and the 48-hour samples of the nylon-based-ADA-benzalkonium chloride material and the nylon-based-ADA-chlorhexidine material all exhibit 100% inhibition against both the Gram-positive bacterium and the Gram-negative bacterium. The freshly prepared sample and the 48-hour sample of the nylon-based-ADA-phenyl biguanide (1-(3-chlorophenyl) biguanide hydrochloride) both exhibit 97% inhibition against the Gram-positive bacterium and respectively 97% and 96% inhibition against the Gram-negative bacterium. It can also be seen that all the nylon-based-ADA-modified antimicrobial materials of the present invention, after having been immersed in water for 48 hours, maintain an antibacterial activity almost the same as that of the freshly prepared materials.

Table 3 Test results for antibacterial activity of the nylon-based-ADA-modified antimicrobial materials

| Samples | Antibacterial activity of freshly prepared samples against the Gram+ bacterium (%) | Antibacterial activity of 48- hour samples against the Gram+ bacterium (%) | Antibacterial activity of freshly prepared samples against the Gram-bacterium (%) | Antibacterial activity of 48-hour samples against the Gram- bacterium (%) |
|---|---|---|---|---|
| Nylon | 5 | 5 | 1 | 1 |
| Nylon-based-ADA | 37 | 36 | 20 | 18 |
| Nylon-based-ADA-cysteine | 95 | 95 | 95 | 94 |
| Nylon-based-ADA-lysine | 91 | 90 | 88 | 87 |
| Nylon-based-ADA-benzalkonium chloride | 100 | 100 | 100 | 100 |

(continued)

| Samples | Antibacterial activity of freshly prepared samples against the Gram+ bacterium (%) | Antibacterial activity of 48- hour samples against the Gram+ bacterium (%) | Antibacterial activity of freshly prepared samples against the Gram-bacterium (%) | Antibacterial activity of 48-hour samples against the Gram-bacterium (%) |
|---|---|---|---|---|
| Nylon-based-ADA-chlorhexidine | 100 | 100 | 100 | 100 |
| Nylon-based-ADA-phenyl biguanide | 97 | 97 | 97 | 96 |

**Example 3b: Determination of antibacterial activity of the ADA-modified antimicrobial materials based on ISO 20743:2013**

In this example, the polypropylene-based-ADA-modified antimicrobial materials and the cellulose-based-ADA-modified antimicrobial materials prepared in Example 1 were tested for their antibacterial activity. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

I. Test methods

Gram-positive *Staphylococcus aureus* and Gram negative *Klebsiella pneumonia* were taken as bacteria examples. The polypropylene-based-ADA-modified antimicrobial materials and the cellulose-based-ADA-modified antimicrobial materials were tested for their antibacterial activity based on "ISO 20743:2013 Textiles-Determination of antibacterial activity of textile products". Log values of bacterial counts in the *Staphylococcus aureus* and *Klebsiella pneumoniae* samples were 5.9 log and 7.3 log respectively.

II. Test results

1. Polypropylene-based-ADA-modified antimicrobial materials

Results for the antibacterial activity of the polypropylene-based-ADA-modified antimicrobial materials against the Gram-positive *Staphylococcus aureus* and Gram negative *Klebsiella pneumonia* are shown in Tables 4a and 4b.
In the case of the Gram-positive *Staphylococcus aureus* (Table 4a), log values of bacterial counts in the primary bacteria sample, the sample treated using the modified material polypropylene-based-ADA-benzalkonium chloride, and the sample treated using the modified material polypropylene-based-ADA-chlorhexidine are 5.9 log, 1.9 log, and 2.1 log, respectively. In other words, the treatment using the modified material polypropylene-based-ADA-benzalkonium chloride achieves a 4.0-log reduction of *Staphylococcus aureus* over the primary bacteria sample, and the treatment using the modified material polypropylene-based-ADA-chlorhexidine achieves a 3.8-log reduction of *Staphylococcus aureus* over the primary bacteria sample.

Table 4a Test results for antibacterial activity of polypropylene-based-ADA-modified antimicrobial materials (against *Staphylococcus aureus*)

| Samples | Bacterial counts- primary sample (Log) | Bacterial counts- treated sample (Log) | Reduction in bacterial counts (Log) |
|---|---|---|---|
| Control | 5.9 | 5.9 | 0 |
| Polypropylene-based-ADA-benzalkonium chloride | 5.9 | 1.9 | 4 |
| Polypropylene-based-ADA-chlorhexidine | 5.9 | 2.1 | 3.8 |

In the case of the Gram-positive *Klebsiella pneumoniae* (Table 4b), log values of bacterial counts in the primary bacteria sample, the sample treated using the modified material polypropylene-based-ADA-benzalkonium chloride, and the sample treated using the modified material polypropylene-based-ADA-chlorhexidine are 7.3 log, 3.1 log, and 3.6 log, respectively. The treatment using the modified material polypropylene-based-ADA-benzalkonium chloride achieves a 4.2-log reduction of *Klebsiella pneumoniae* over the primary bacteria sample, and the treatment using the modified material polypropylene-based-ADA-chlorhexidine achieves a 3.7-log reduction of *Klebsiella pneumonia* over the primary bacteria sample.

Table 4b Test results for antibacterial activity of polypropylene-based-ADA-modified antimicrobial materials (against *Klebsiella pneumonia*)

| Samples | Bacterial counts- primary sample (Log) | Bacterial counts- treated sample (Log) | Reduction in bacterial counts (Log) |
|---|---|---|---|
| Control | 7.3 | 7.3 | 0 |
| Polypropylene-based-ADA-benzalkonium chloride | 7.3 | 3.1 | 4.2 |
| Polypropylene-based-ADA-chlorhexidine | 7.3 | 3.6 | 3.7 |

2. Cellulose-based-ADA-modified antimicrobial materials

Results for the antibacterial activity of the cellulose-based-ADA-modified antimicrobial materials against the Gram-positive *Staphylococcus aureus* and Gram negative *Klebsiella pneumonia* are shown in Tables 5a and 5b.

In the case of the Gram-positive *Staphylococcus aureus* (Table 5a), log values of bacterial counts in the primary bacteria sample, the sample treated using the modified material cellulose-based-ADA-benzalkonium chloride, and the sample treated using the modified material cellulose-based-ADA-chlorhexidine are 5.9 log, 1.7 log, and 1.9 log, respectively. The treatment using the modified material cellulose-based-ADA-benzalkonium chloride achieves a 4.2-log reduction of *Staphylococcus aureus* over the primary bacteria sample, and the treatment using the modified material cellulose-based-ADA-chlorhexidine achieves a 4.0-log reduction of *Staphylococcus aureus* over the primary bacteria sample.

In the case of the Gram-positive *Klebsiella pneumoniae* (Table 5b), log values of bacterial counts in the primary bacteria sample, the sample treated using the modified material cellulose-based-ADA-benzalkonium chloride, and the sample treated using the modified material cellulose-based-ADA-chlorhexidine are 7.3 log, 2.9 log, and 3.5 log, respectively. The treatment using the modified material cellulose-based-ADA-benzalkonium chloride achieves a 4.4-log reduction of *Klebsiella pneumoniae* over the primary bacteria sample, and the treatment using the modified material cellulose-based-ADA-chlorhexidine achieves a 3.8-log reduction of *Klebsiella pneumoniae* over the primary bacteria sample.

Table 5a Test results for antibacterial activity of cellulose-based-ADA-modified antimicrobial materials (against *Staphylococcus aureus*)

| Samples | Bacterial counts- primary sample (Log) | Bacterial counts- treated sample (Log) | Reduction in bacterial counts (Log) |
|---|---|---|---|
| Control | 5.9 | 5.9 | 0 |
| Cellulose-based-ADA-benzalkonium chloride | 5.9 | 1.7 | 4.2 |
| Cellulose-based-ADA- chlorhexidine | 5.9 | 1.9 | 4 |

Table 5b Test results for antibacterial activity of cellulose-based-ADA-modified antimicrobial materials (against *Klebsiella pneumoniae*)

| Samples | Bacterial counts- primary sample (Log) | Bacterial counts- treated sample (Log) | Reduction in bacterial counts (Log) |
|---|---|---|---|
| Control | 7.3 | 7.3 | 0 |
| Cellulose-based-ADA-benzalkonium chloride | 7.3 | 2.9 | 4.4 |

(continued)

| Table 5b Test results for antibacterial activity of cellulose-based-ADA-modified antimicrobial materials (against *Klebsiella pneumoniae*) | | | |
|---|---|---|---|
| Samples | Bacterial counts- primary sample (Log) | Bacterial counts- treated sample (Log) | Reduction in bacterial counts (Log) |
| Cellulose-based-ADA- chlorhexidine | 7.3 | 3.5 | 3.8 |

**Example 4: Determination of antifungal activity of the ADA-modified antimicrobial materials**

In this example, the polypropylene-based-ADA-modified antimicrobial materials and the nylon-based-ADA-modified antimicrobial materials prepared in Example 1 were tested for their antifungal activity. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

I. Test methods

*Saccharomyces cerevisiae* and *Aspergillus Niger* were taken as fungi examples. The polypropylene-based-ADA-modified antimicrobial materials, the cellulose-based-ADA-modified antimicrobial materials, and the nylon-based-ADA-modified antimicrobial materials were tested for their antifungal activity. Each of the samples was provided in triplicate, and an average value was taken as a result.

*Saccharomyces cerevisiae* and *Aspergillus Niger* were incubated in a yeast peptone dextrose (YPD) medium overnight at 30°C under stirring. The resulting fresh fungal culture was diluted into a suspension with a number of colonies of $10^{-4}$ CFU/ml. 0.1 g of a polymer-ADA-modified antimicrobial material and 10 mL of the fungal suspension were mixed and placed in a flask for incubation at 37°C for 2 hours in a shaking bed at a shaking speed of 120 rpm. Another 10 mL of the fungal suspension was separately placed in another flask as a control. After the incubation, 10-100 $\mu$l of the fungal suspension was seeded onto an YPD agar plate and incubated at 30°C for 18-36 hours. After 36 hours of incubation, the number of viable colonies was calculated using the plate count method.

The antifungal activity was calculated based on the following formula:

$$I = \frac{N_0 - N_i}{N_0} \times 100\%$$

where I represents the antifungal activity, $N_0$ represents the number of colonies after the incubation in the control group, and $N_i$ represents the number of colonies after incubation in the treatment group.

In addition, in order to check the stability of the antifungal coating, all the polymer-ADA-modified antimicrobial materials were immersed in autoclaved Milli Q ultra-pure water for 48 hours, and their antifungal activity was compared with that of the freshly prepared materials.

II. Test results

1. Polypropylene-based-ADA-modified antimicrobial materials

Results for the antifungal activity of the polypropylene-based-ADA-modified antimicrobial material against *Saccharomyces cerevisiae* and *Aspergillus Niger* are shown in Table 4. The polypropylene material alone has an antifungal activity of only 15%. The freshly prepared sample and the 48-hour sample of the polypropylene-based-ADA material exhibit respectively 35% and 32% inhibition against *Saccharomyces cerevisiae* and *Aspergillus Niger.* The freshly prepared sample and the 48-hour sample of the polypropylene-based-ADA-cysteine material exhibit respectively 70% and 68% inhibition against *Saccharomyces cerevisiae* and *Aspergillus Niger.* The freshly prepared sample and the 48-hour sample of the polypropylene-based-ADA-lysine material both exhibit 73% inhibition against *Saccharomyces cerevisiae* and *Aspergillus Niger.* The freshly prepared samples and the 48-hour samples of the polypropylene-based-ADA-benzalkonium chloride material, the polypropylene-based-ADA-chlorhexidine material, and the polypropylene-based-ADA-phenyl biguanide (1-(3-chlorophenyl) biguanide hydrochloride) all exhibit 100% inhibition against *Saccharomyces cerevisiae* and *Aspergillus Niger.* It can also be seen that all the polypropylene-based-ADA-modified antimicrobial materials of the present invention, after having been immersed in water for 48 hours, maintain an antifungal activity almost the same as that of the freshly prepared materials.

Table 6 Test results for antifungal activity of the polypropylene-based-ADA-modified antimicrobial materials

| Samples | Antifungal activity of freshly prepared samples (%) | Antifungal activity of 48-hour samples (%) |
|---|---|---|
| Polypropylene | 15 | 15 |
| Polypropylene- based-ADA | 35 | 32 |
| Polypropylene- based-ADA-cysteine | 70 | 68 |
| Polypropylene - ADA-lysine | 73 | 73 |
| Polypropylene- based-ADA-benzalkonium chloride | 100 | 100 |
| Polypropylene- based-ADA-chlorhexidine | 100 | 100 |
| Polypropylene- based-ADA-phenyl biguanide | 100 | 100 |

2. Cellulose-based-ADA-modified antimicrobial materials

Results for the antifungal activity of the cellulose-based-ADA-modified antimicrobial materials against *Saccharomyces cerevisiae* and *Aspergillus Niger* are shown in Table 5. The cellulose material alone has an antifungal activity of only 23%. The freshly prepared sample and the 48-hour sample of the cellulose-based-ADA material exhibit respectively 37% and 36% inhibition against *Saccharomyces cerevisiae* and *Aspergillus Niger.* The freshly prepared sample and the 48-hour sample of the cellulose-based-ADA-cysteine material both exhibit 79% inhibition against *Saccharomyces cerevisiae* and *Aspergillus Niger.* The freshly prepared sample and the 48-hour sample of the cellulose-based-ADA-lysine material exhibit respectively 85% and 84% inhibition against *Saccharomyces cerevisiae* and *Aspergillus Niger.* The freshly prepared samples and the 48-hour samples of the cellulose-based-ADA-benzalkonium chloride material, the cellulose-based-ADA-chlorhexidine material, and the cellulose-based-ADA-phenyl biguanide (1-(3-chlorophenyl) biguanide hydrochloride) all exhibit 100% inhibition against *Saccharomyces cerevisiae* and *Aspergillus Niger.* It can also be seen that all the cellulose-based-ADA-modified antimicrobial materials of the present invention, after having been immersed in water for 48 hours, maintain an antifungal activity almost the same as that of the freshly prepared materials.

Table 7 Test results for antifungal activity of the cellulose-based-ADA-modified antimicrobial materials

| Samples | Antifungal activity of freshly prepared samples (%) | Antifungal activity of 48-hour samples (%) |
|---|---|---|
| Cellulose | 23 | 21 |
| Cellulose-based- ADA | 37 | 36 |
| Cellulose-based- ADA-cysteine | 79 | 79 |
| Cellulose-based- ADA-lysine | 85 | 84 |
| Cellulose-based- ADA-benzalkonium chloride | 100 | 100 |
| Cellulose-based- ADA-chlorhexidine | 100 | 100 |
| Cellulose-based- ADA-phenyl biguanide | 100 | 100 |

3. Nylon-based-ADA-modified antimicrobial materials

Results for the antifungal activity of the nylon-based-ADA-modified antimicrobial materials against *Saccharomyces cerevisiae* and *Aspergillus Niger* are shown in Table 6. The nylon material alone has an antifungal activity of only 18%. The freshly prepared sample and the 48-hour sample of the nylon-based-ADA material exhibit respectively 40% and 39% inhibition against the *Saccharomyces cerevisiae* and *Aspergillus Niger.* The freshly prepared sample and the 48-hour sample of the nylon-based-ADA-cysteine material exhibit respectively 75% and 70% inhibition against *Saccharomyces cerevisiae* and *Aspergillus Niger.* The freshly prepared sample and the 48-hour sample of the nylon-based-ADA-lysine material both exhibit 82% inhibition against *Saccharomyces cerevisiae* and *Aspergillus Niger.* The freshly prepared samples and the 48-hour samples of the nylon-based-ADA-benzalkonium chloride material, the nylon-based-ADA-chlorhexidine material, and the nylon-based-ADA-phenyl biguanide (1-(3-chlorophenyl) biguanide hydrochloride) material all exhibit 100% inhibition against *Saccharomyces cerevisiae* and *Aspergillus Niger.* It can also be seen that all the nylon-based-ADA-modified antimicrobial materials of the present invention, after having been immersed in water for 48 hours, maintain an antifungal activity almost the same as that of the freshly prepared materials.

Table 8 Test results for antifungal activity of the nylon-based-ADA-modified antimicrobial materials

| Samples | Antifungal activity of freshly prepared samples (%) | Antifungal activity of 48-hour samples (%) |
|---|---|---|
| Nylon | 18 | 17 |
| Nylon-based-ADA | 40 | 39 |
| Nylon-based-ADA-cysteine | 75 | 70 |
| Nylon-based-ADA-lysine | 82 | 82 |
| Nylon-based-ADA-benzalkonium chloride | 100 | 100 |
| Nylon-based-ADA-chlorhexidine | 100 | 100 |
| Nylon-based-ADA-phenyl biguanide | 100 | 100 |

**Example 5: Determination of antiviral activity of ADA-modified antimicrobial materials**

In this example, the polypropylene-based-ADA-modified antimicrobial materials and the nylon-based-ADA-modified antimicrobial materials prepared in Example 1 were tested for their antiviral activity. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

I. Test methods

The novel coronavirus (SARS-CoV-2) was taken as a virus example. The polypropylene-based-ADA-modified antimicrobial materials and the nylon-based-ADA-modified antimicrobial materials were tested for their antiviral activity. Each of the samples was provided in triplicate, and an average value was taken as a result.

The material was dried in a biological safety cabinet, and then cut into pieces of a size of about 0.3 cm x 0.3 cm. 200 $\mu$l of a suspension of the virus (SARS-CoV-2) was measured and mixed with the material in a 2-mL sterile centrifuge tube, and then incubated at room temperature for 1 hour. An equivalent amount of the virus sample (the suspension of the virus) was separately taken and placed in another sterile centrifuge tube as a control. For the polypropylene-based-ADA-modified antimicrobial materials and the nylon-based-ADA-modified antimicrobial materials, the initial Log TCID50/mL of the virus samples are 7.2 log and 6.9 log, respectively. After the incubation, the virus was eluted with 800 $\mu$l of PBS, and 400 $\mu$l of the eluate was collected from a Micro Spin S-400 HR column (GE Healthcare) to remove eluted active ingredients and reduce cytotoxicity. 50% tissue culture infective dose (TCID 50) was used to titrate the virus eluate in the control group and the residual virus eluate in the treated group. The virus titer was calculated using the Reed-Muench method.

II. Test results

1. Polypropylene-based-ADA-modified antimicrobial materials

Results for the antiviral activity of the polypropylene-based-ADA-modified antimicrobial materials against the novel coronavirus SARS-CoV-2 are shown in Table 7. Log TCID50/mL of the virus sample in the control group and the virus samples treated using the polypropylene material, the polypropylene-based-ADA material, the modified material polypropylene-based-ADA-benzalkonium chloride and the modified material polypropylene-based-ADA-chlorhexidine are 7.2 log, 6.7 log, 6.73 log, ≤1.3 log, and 5.03 log, respectively. The modified material polypropylene-based-ADA-benzalkonium chloride reduces the virus titer by ≥5.9 log relative to that in the control sample, and reduces the virus titer by ≥5.4 log relative to those in the samples treated using the polypropylene material and the polypropylene-based-ADA material. The modified material polypropylene-based-ADA-chlorhexidine reduces the virus titer by 2.7 log relative to that in the control sample, and reduces the virus titer by 1.67 log relative to those in the samples treated using the polypropylene material and the polypropylene-based-ADA material.

The polypropylene material and the polypropylene-based-ADA material reduce the virus titer to some extent, but the fact is that they adsorb the viruses on the surfaces of the materials so that the viruses are not eluted, and they do not kill the viruses. In contrast, the modified material polypropylene-based-ADA-benzalkonium chloride and the modified material polypropylene-based-ADA-chlorhexidine directly kill the viruses through benzalkonium chloride and chlorhexidine to thereby achieve a 100% antiviral effect.

Table 9 Test results for antiviral activity of the polypropylene-based-ADA-modified antimicrobial materials (against SARS-CoV-2)

| Samples | Log TCID50/mL | Virus Titer of eluate | Virus content in eluate (% of the control virus sample) | Virus Reduction (% of the control virus sample) | Virus Reduction (Log) |
|---|---|---|---|---|---|
| Control virus sample | 7.2 | 15848931.92 | 100 | 0 | 0 |
| polyethylene | 6.7 | 5011872.33 | 31.62 | 68.38 | 0.5 |
| Polypropylene-based-ADA | 6.73 | 5436183.64 | 34.3 | 65.7 | 0.47 |
| Polypropylene-based- ADA-benzalkonium chloride | ≤1.3 | ≦ 19.95 | 0.00012 | 99.99 | 5.9 |
| Polypropylene-based- ADA-chlorhexidine | 5.03 | 107151.93 | 0.67 | 99.33 | 2.17 |

2. Nylon-based-ADA-modified antimicrobial materials

Results for the antiviral activity of the nylon-based-ADA-modified antimicrobial materials against the novel coronavirus SARS-CoV-2 are shown in Table 8. Log TCID50/mL of the virus sample in the control group and the virus samples treated using the nylon material, the nylon-based-ADA material, the nylon-based-ADA-cysteine material, the nylon-based-ADA-lysine material, the nylon-based-ADA-benzalkonium chloride material and the nylon-based-ADA-chlorhexidine material are 6.9 log, 5.47 log, 5.53 log, ≤2.2 log, and 5.37 log, respectively. The nylon-based-ADA-benzalkonium chloride material reduces the virus titer by ≥4.7 log, ≥3.27 log, and ≥3.33 log respectively relative to that in the control sample and those in the samples treated using the nylon material and the nylon-based-ADA material. The nylon-based-ADA-chlorhexidine material reduces the virus titer by 1.53 log, 0.1 log, and 0.16 log respectively relative to that in the control sample and those in the samples treated using the nylon material and the nylon-based-ADA material.

The nylon material and the nylon-based-ADA material can reduce the virus titer, but the truth is that they adsorb the viruses on the surfaces of the materials and do not kill the viruses. In contrast, the nylon-based-ADA-benzalkonium chloride material and the nylon-based-ADA-chlorhexidine material directly kill the viruses through benzalkonium chloride and chlorhexidine to thereby achieve an antiviral effect.

Table 10 Test results for antiviral activity of the nylon-based-ADA-modified antimicrobial materials (against SARS-CoV-2)

| Samples | Log TCID50/mL | Virus Titer of eluate | Virus content in eluate (% of the control virus sample) | Virus Reduction (% of the control virus sample) | Virus Reduction (Log) |
|---|---|---|---|---|---|
| Control virus sample | 6.9 | 7943282.35 | 100 | 0 | 0 |
| Nylon | 5.47 | 295120.92 | 3.7 | 96.3 | 1.47 |
| Nylon-based-ADA | 5.53 | 338844.156 | 4.26 | 95.74 | 1.37 |
| Nylon-based-ADA cysteine | 6.2 | 1584893.19 | 19 | 81 | 0.7 |
| Nylon-based-ADA-lysine | 6.2 | 1584893.19 | 19 | 81 | 0.7 |

(continued)

| Samples | Log TCID50/mL | Virus Titer of eluate | Virus content in eluate (% of the control virus sample) | Virus Reduction (% of the control virus sample) | Virus Reduction (Log) |
|---|---|---|---|---|---|
| Nylon-based-ADA-benzalkonium chloride | ≤2.2 | ≤158.48 | .002 | 99.99 | 4.7 |
| Nylon-based-ADA-chlorhexidine | 5.37 | 234422.882 | 2.95 | 97.01 | 1.53 |

**Example 5b: Determination of antiviral activity of the ADA-modified antimicrobial materials based on ISO 18184:2019 (E)**

In this example, the polypropylene-based-ADA-modified antimicrobial materials and the cellulose-based-ADA-modified antimicrobial materials prepared in Example 1 were tested for their antiviral activity. This example took the ADA-modified antimicrobial materials prepared by method I for preparing an ADA-functionalized material and method I for preparing an ADA-modified antimicrobial material used in Example 1 as an example. Highly similar results were also obtained for the ADA-modified antimicrobial materials prepared by other methods used in Example 1.

1. Test methods

The influenza virus H1N1 was taken as a virus example. The polypropylene-based-ADA-modified antimicrobial materials and the ellulose-based-ADA-modified antimicrobial materials were tested for their antiviral activity based on "ISO 18184:2019 Textiles-Determination of antiviral activity of textile products". For the polypropylene-based-ADA-modified antimicrobial materials and the cellulose-based-ADA-antimicrobial materials, the initial LogTCID50/ml of the primary virus sample used was 6.85 log.

II. Test results

1. Polypropylene-based-ADA-modified antimicrobial materials

Results for the antiviral activity of the polypropylene-based-ADA-modified antimicrobial materials against H1N1 are shown in Table 9.
The Log TCID50/mL in the primary virus sample and in the samples treated using the modified material polypropylene-based-ADA-benzalkonium chloride and the modified material polypropylene-based-ADA-chlorhexidine are 6.85 log, 1.5 log, and 3.5 log, respectively. The polypropylene-based-ADA-benzalkonium chloride material reduces the virus titer by 5.35 log relative to that in the primary virus sample. The polypropylene-based-ADA-chlorhexidine material reduces the virus titer by 3.35 log relative to that in the primary virus sample.

Table 11 Test results for antiviral activity of the polypropylene-based-ADA-modified antimicrobial materials (against H1NI Influenza Virus)

| Samples | Virus tier-primary virus sample (Log) | Virus tier-treated samples (Log) | Virus reduction (Log) |
|---|---|---|---|
| Control sample | 6.85 | 6.85 | 0 |
| Polypropylene-based-ADA-benzalkonium chloride | 6.85 | 1.5 | 5.35 |
| Polypropylene-based-ADA-chlorhexidine | 6.85 | 3.5 | 3.35 |

2. Cellulose-based-ADA-modified antimicrobial materials

Results of the antiviral activity of the cellulose-based-ADA-modified antimicrobial materials against H1N1 are shown in Table 8.
The Log TCID50/mL in the primary virus sample and in the samples treated using the modified material poly-

EP 4 190 848 A1

propylene-based-ADA-benzalkonium chloride and the modified material polypropylene-based-ADA-chlorhexidine are 6.85 log, 1.2 log, and 3.7 log, respectively. The cellulose-based-ADA-benzalkonium chloride material reduces the virus titer by 5.65 log relative to that in the primary virus sample. The cellulose-based-ADA-chlorhexidine material reduces the virus titer by 3.15 log relative to that in the primary virus sample.

Table 12 Test results for antiviral activity of the cellulose-based-ADA-modified antimicrobial materials (against H1NI influenza virus)

| Samples | Virus tier-primary virus sample (Log) | Virus tier-treated samples (Log) | Virus reduction (Log) |
|---|---|---|---|
| Control sample | 6.85 | 6.85 | 0 |
| Cellulose-based-ADA-benzalkonium chloride | 6.85 | 1.2 | 5.65 |
| Cellulose-based-ADA-chlorhexidine | 6.85 | 3.7 | 3.15 |

The above embodiments only represent several embodiments of the present invention which involve specific and detailed description and should not be interpreted as a limitation on the scope of the present invention. It should be noted that for those skilled in the art, various modifications and improvements can be made without departing from the concept of the present invention, and such modifications and improvements should all fall within the protection scope of the present invention. The protection scope of the present invention should be subject to the appended claims.

**Claims**

1.  An alginate dialdehyde-functionalized material, comprising:

    a support layer, which is formed of one or more porous or non-porous materials; and
    a link layer, which is formed of alginate dialdehyde, wherein the alginate dialdehyde is linked to the support layer through covalent bonding, electrostatic adsorption, hydrogen bonding, or any combination thereof;
    wherein the link layer is capable of combining with a functional material through covalent bonding, electrostatic adsorption, hydrophilic adsorption, hydrophobic adsorption, hydrogen bonding, or any combination thereof.

2.  The functionalized material according to claim 1, wherein the one or more porous or non-porous materials are one or more selected from polymers, composite materials, and combinations thereof.

3.  The functionalized material according to claim 2, wherein the polymer or the composite material is selected from the group consisting of polypropylene, cellulose, regenerated cellulose, polyvinylidene fluoride, polyethersulfone, polystyrene, polytetrafluoroethylene, polyethylene, polyimide, polyamide, and combinations thereof.

4.  The functionalized material according to any one of claims 1 to 3, wherein a mass ratio of the link layer to the support layer is 1:100 to 1:10.

5.  The functionalized material according to claim 4, wherein the mass ratio of the link layer to the support layer is 1:50 to 1:20.

6.  The functionalized material according to any one of claims 1 to 3, wherein the functional material is an antimicrobial material capable of inhibiting microorganisms, the microorganisms being selected from the group consisting of bacteria, fungi, viruses, and combinations thereof, wherein: the bacteria are selected from the group consisting of *Chlamydia pneumoniae, Streptococcus pneumoniae, Mycobacterium tuberculosis, Streptococcus Group A, Corynebacterium diphtheriae, Hemophilus influenzae, Neisseria meningitidis, Clostridium difficile,* Methicillin-resistant *Staphylococcus aureus,* Vancomycin-resistant *Enterococci, Acinetobacter baumannii,* and combinations thereof; the fungi are selected from the group consisting *of Pneumocystis, Aspergillus, Coccidioides, Blastomyces, Candida, Mucor, Sporothrix, Epidermophyton,* and combinations thereof; and the viruses are selected from the group consisting of respiratory syncytial virus, hepatitis virus, varicella virus, polio virus, variola virus, measles virus, mumps virus, chlamydia trachomatis, influenza virus, SARS-CoV virus, SARS-CoV-2 virus, H1N1 virus, H5N1 virus, H5N7 virus, MERS-CoV virus, *Staphylococcus aureus, Klebsiella pneumonia, Aspergillus Niger,* Ebola virus, and combinations thereof.

7. The functionalized material according to claim 6, wherein the antimicrobial material is selected from the group consisting of amino acids, quaternary ammonium compounds, chlorhexidine compounds, alexidine compounds, biguanide compounds, and combinations thereof, wherein: the amino acid is selected from the group consisting of cysteine, tyrosine, lysine, arginine, aspartic acid, and combinations thereof; the quaternary ammonium compound is selected from the group consisting of alkyl dimethyl benzyl ammonium chloride, alkyl didecyl dimethyl ammonium chloride, dialkyl dimethyl ammonium chloride, dialkyl quaternary ammonium salts, and combinations thereof; the chlorhexidine compound is chlorhexidine digluconate and/or chlorhexidine dihydrochloride; the alexidine compound is alexidine dihydrochloride; and the biguanide compound is 1-(3-chlorophenyl) biguanide hydrochloride.

8. An alginate dialdehyde-modified material, comprising:

the alginate dialdehyde-functionalized material according to any one of claims 1 to 5; and
a functional layer, which is formed of a functional material capable of being linked to the link layer through covalent bonding, electrostatic adsorption, hydrophilic adsorption, hydrophobic adsorption, hydrogen bonding, or any combination thereof.

9. The modified material according to claim 8, wherein the functional material is an antimicrobial material, and the alginate dialdehyde-modified material is an alginate dialdehyde-modified antimicrobial material.

10. The modified material according to claim 9, wherein the antimicrobial material is selected from the group consisting of amino acids, quaternary ammonium compounds, chlorhexidine compounds, alexidine compounds, biguanide compounds, and combinations thereof.

11. The modified material according to claim 10, wherein the amino acid is selected from the group consisting of cysteine, tyrosine, lysine, arginine, aspartic acid, and combinations thereof.

12. The modified material according to claim 10, wherein the quaternary ammonium compound is selected from the group consisting of alkyl dimethyl benzyl ammonium chloride, alkyl didecyl dimethyl ammonium chloride, dialkyl dimethyl ammonium chloride, dialkyl quaternary ammonium salts, and combinations thereof.

13. The modified material according to claim 10, wherein the chlorhexidine compound is chlorhexidine digluconate and/or chlorhexidine dihydrochloride; the alexidine compound is alexidine dihydrochloride; and the biguanide compound is 1-(3-chlorophenyl) biguanide hydrochloride.

14. The modified material according to claim 9, wherein the antimicrobial material and the alginate dialdehyde-modified antimicrobial material each have an antimicrobial activity against bacteria, fungi, viruses, and combinations thereof, wherein the bacteria are selected from the group consisting of *Chlamydia pneumoniae, Streptococcus pneumoniae, Mycobacterium tuberculosis, Streptococcus Group A, Corynebacterium diphtheriae, Hemophilus influenzae, Neisseria meningitidis, Clostridium difficile,* Methicillin-resistant *Staphylococcus aureus,* Vancomycin-resistant *Enterococci, Acinetobacter baumannii,* and combinations thereof; the fungi are selected from the group consisting of *Pneumocystis, Aspergillus, Coccidioides, Blastomyces, Candida, Mucor, Sporothrix, Epidermophyton,* and combinations thereof; and the viruses are selected from the group consisting of respiratory syncytial virus, hepatitis virus, varicella virus, polio virus, variola virus, measles virus, mumps virus, chlamydia trachomatis, influenza virus, SARS-CoV virus, SARS-CoV-2 virus, H1N1 virus, H5N1 virus, H5N7 virus, MERS-CoV virus, *Staphylococcus aureus, Klebsiella pneumonia, Aspergillus Niger,* Ebola virus, and combinations thereof.

15. The modified material according to claim 9, wherein the alginate dialdehyde-modified antimicrobial material is an alginate dialdehyde-modified antibacterial material selected from the group consisting of polypropylene-based-alginate dialdehyde-cysteine, polypropylene-based-alginate dialdehyde-lysine, polypropylene-based-alginate dialdehyde-benzalkonium chloride, polypropylene-based alginate dialdehyde-chlorhexidine, polypropylene-based-alginate dialdehyde-phenyl biguanide, cellulose-based-alginate dialdehyde-cysteine, cellulose-based-alginate dialdehyde aldehydes-lysine, cellulose-based-alginate dialdehyde-benzalkonium chloride, cellulose-based-alginate dialdehyde-chlorhexidine, cellulose-based-alginate dialdehyde-phenyl biguanide, nylon-based-alginate dialdehyde-cysteine, nylon-based-alginate dialdehyde-lysine, nylon-based-alginate dialdehyde-benzalkonium chloride, nylon-based-alginate dialdehyde-chlorhexidine, nylon-based-alginate dialdehyde-phenyl biguanide, and combinations thereof.

16. The modified material according to claim 9, wherein the alginate dialdehyde-modified antimicrobial material is an alginate dialdehyde-modified antifungal material selected from the group consisting of polypropylene-based-alginate dialdehyde-cysteine, polypropylene-based-alginate dialdehyde-lysine, polypropylene-based-alginate dialdehyde-benzalkonium chloride, polypropylene-based-alginate dialdehyde-chlorhexidine, polypropylene-based-alginate dialdehyde-phenyl biguanide, cellulose-based-alginate dialdehyde-cysteine, cellulose-based-alginate dialdehyde-lysine, cellulose-based-alginate dialdehyde-benzalkonium chloride, cellulose-based-alginate dialdehyde-chlorhexidine, cellulose-based-alginate dialdehyde-phenyl biguanide, nylon-based-alginate dialdehyde-cysteine, nylon-based-alginate dialdehyde-lysine, nylon-based-alginate dialdehyde-benzalkonium chloride, nylon-based-alginate dialdehyde-chlorhexidine, nylon-based-alginate dialdehyde-phenyl biguanide, and combinations thereof.

17. The modified material according to claim 9, wherein the alginate dialdehyde-modified antimicrobial material is an alginate dialdehyde-modified antiviral material selected from the group consisting of polypropylene-based-alginate dialdehyde-benzalkonium chloride, polypropylene-based-alginate dialdehyde-chlorhexidine, nylon-based-alginate dialdehyde-cysteine, nylon-based-alginate dialdehyde-lysine, nylon-based-alginate dialdehyde-benzalkonium chloride, nylon-based-alginate dialdehyde-chlorhexidine, and combinations thereof.

18. The modified material according to any one of claims 8 to 17, wherein the functional material in the functional layer accounts for 1-10% of a total mass of the modified material.

19. The modified material according to claim 18, wherein the functional material in the functional layer accounts for 2-5% of the total mass of the modified material.

20. A preparation method of the alginate dialdehyde-functionalized material according to any one of claims 1 to 7, comprising the following steps:

   mixing a porous or non-porous material with an aqueous solution of alginate dialdehyde, and subjecting the resulting mixture to a reaction at a temperature of 10-100°C and a pH value of 1.5-8 for 10 minutes to 24 hours under agitating at a speed of 60-200 rpm;
   or,
   directly applying or spraying an aqueous solution of alginate dialdehyde with a pH value of 1.5-8 to a surface of a porous or non-porous material, followed by drying at a temperature of 10-100°C for 10 minutes to 24 hours, to obtain the alginate dialdehyde-functionalized material.

21. The preparation method according to claim 20, wherein a mass ratio of the alginate dialdehyde contained in the aqueous solution of alginate dialdehyde to the porous or non-porous material is 1: 100 to 1:10.

22. A preparation method of the alginate dialdehyde-modified material according to any one of claims 8 to 19, comprising the following steps:

   adding the alginate dialdehyde-functionalized material according to any one of claims 1 to 7 or the alginate dialdehyde-functionalized material prepared by the method according to claim 20 or 21 to an aqueous solution of a functional material, and subjecting the resulting mixture to a reaction at a temperature of 10-100°C and a pH value of 1.5-8 for 10 minutes to 24 hours under agitating at a speed of 60-200 rpm;
   or,
   directly applying or spraying an aqueous solution of a functional material with a pH value of 1.5-8 to the alginate dialdehyde-functionalized material, followed by drying at a temperature of 10-100°C for 10 minutes to 24 hours, to obtain the modified material;
   wherein the functional material is capable of being linked to the link layer through covalent bonding, electrostatic adsorption, hydrophilic adsorption, hydrophobic adsorption, hydrogen bonding, or any combination thereof.

23. The preparation method according to claim 22, wherein a mass ratio of the functional material contained in the aqueous solution of the functional material to the alginate dialdehyde-functionalized material is 1: 1000 to 1:10.

24. A preparation method of the alginate dialdehyde-modified material according to any one of claims 8 to 19, comprising the following steps:

   mixing an functional material with an aqueous solution of alginate dialdehyde to obtain an aqueous solution of alginate dialdehyde-functional material; and
   subjecting the resulting aqueous solution and a porous or non-porous material to a reaction at a temperature of 10-100°C and a pH value of 1.5-8 for 10 minutes to 24 hours under agitating at a speed of 60-200 rpm; or, directly applying the resulting aqueous solution with a pH value of 1.5-8 to a surface of a porous or non-porous material, followed by drying at a temperature of 10-100°C for 10 minutes to 24 hours, to obtain the modified material;

wherein the functional material is capable of being linked to the link layer through covalent bonding, electrostatic adsorption, hydrophilic adsorption, hydrophobic adsorption, hydrogen bonding, or any combination thereof.

25. The preparation method according to claim 24, wherein a mass ratio of the functional material to alginate dialdehyde to the porous or non-porous material is (1-10): (10-100): 1000.

26. The preparation method according to any one of claims 20 to 25, further comprising the following steps: washing the resulting product with water, and storing the product in a wet state or drying the product in the air or in an oven.

27. The preparation method according to any one of claims 20 to 25, wherein the porous or non-porous material is one or more selected from polymers, composite materials, and combinations thereof, wherein the polymer or the composite material is selected from the group consisting of polypropylene, cellulose, regenerated cellulose, polyvinylidene fluoride, polyethersulfone, polystyrene, polytetrafluoroethylene, polyethylene, polyimide, polyamide, and combinations thereof.

28. The preparation method according to any one of claims 20 to 25, wherein the functional material is an antimicrobial material capable of inhibiting microorganisms, the microorganisms being selected from the group consisting of bacteria, fungi, viruses, and combinations thereof, wherein: the bacteria are selected from the group consisting of *Chlamydia pneumoniae, Streptococcus pneumoniae, Mycobacterium tuberculosis, Streptococcus Group A, Corynebacterium diphtheriae, Hemophilus influenzae, Neisseria meningitidis, Clostridium difficile,* Methicillin-resistant *Staphylococcus aureus,* Vancomycin-resistant *Enterococci, Acinetobacter baumannii,* and combinations thereof; the fungi are selected from the group consisting *of Pneumocystis, Aspergillus, Coccidioides, Blastomyces, Candida, Mucor, Sporothrix, Epidermophyton,* and combinations thereof; and the viruses are selected from the group consisting of respiratory syncytial virus, hepatitis virus, varicella virus, polio virus, variola virus, measles virus, mumps virus, chlamydia trachomatis, influenza virus, SARS-CoV virus, SARS-CoV-2 virus, H1N1 virus, H5N1 virus, H5N7 virus, MERS-CoV virus, *Staphylococcus aureus, Klebsiella pneumonia, Aspergillus Niger,* Ebola virus, and combinations thereof.

29. The preparation method according to any one of claims 20 to 25, wherein the functional material is an antimicrobial material, the antimicrobial material being selected from the group consisting of amino acids, quaternary ammonium compounds, chlorhexidine compounds, alexidine compounds, biguanide compounds, and combinations thereof, wherein: the amino acid is selected from the group consisting of cysteine, tyrosine, lysine, arginine, aspartic acid, and combinations thereof; the quaternary ammonium compound is selected from the group consisting of alkyl dimethyl benzyl ammonium chloride, alkyl didecyl dimethyl ammonium chloride, dialkyl dimethyl ammonium chloride, dialkyl quaternary ammonium salts, and combinations thereof; the chlorhexidine compound is chlorhexidine digluconate and/or chlorhexidine dihydrochloride; the alexidine compound is alexidine dihydrochloride; and the biguanide compound is 1-(3-chlorophenyl) biguanide hydrochloride.

30. Use of the alginate dialdehyde-functionalized material according to any one of claims 1 to 7, the alginate dialdehyde-modified material according to any one of claims 8 to 19, the preparation method according to any one of claims 20 to 29, or the material prepared according to the preparation method according to any one of claims 20 to 29 in the manufacture of medical protective products and other antimicrobial products.

31. The use according to claim 30, comprising the manufacture of masks, isolation gowns, gloves, personal protective articles, bandages, medical tapes, medical caps, medical bed sheets, medical clothes, air filters, water filters, electronic products, household appliances, and vehicle parts.

Fig. 1

Fig. 2

Fig. 3

## INTERNATIONAL SEARCH REPORT

| International application No. |
| --- |
| **PCT/CN2021/116582** |

**A. CLASSIFICATION OF SUBJECT MATTER**

C08J 9/42(2006.01)i; C08J 7/12(2006.01)i; C08L 5/04(2006.01)i; C08J 5/18(2006.01)i

According to International Patent Classification (IPC) or to both national classification and IPC

**B. FIELDS SEARCHED**

Minimum documentation searched (classification system followed by classification symbols)

C08J; C08L

Documentation searched other than minimum documentation to the extent that such documents are included in the fields searched

Electronic data base consulted during the international search (name of data base and, where practicable, search terms used)

CNABS; CNTXT; CJFD; VEN; USTXT; EPTXT; WOTXT; CNKI; 万方; 读秀; ISI_Web of Science: 牛津大学（苏州）科技有限公司, 王丹丹, 海藻酸二醛, 氧化海藻酸钠, 抗菌, 抗微生物, 防污, 氨基酸, 氯己定, alginate dialdehyde, oxidated sodium alginate, anti+, antibacterial, antifouling, biofouling, grafting, Chlorhexidine, amino acid

**C. DOCUMENTS CONSIDERED TO BE RELEVANT**

| Category* | Citation of document, with indication, where appropriate, of the relevant passages | Relevant to claim No. |
| --- | --- | --- |
| X | Rashid Khan et al. "Surface Grafting of Reverse Osmosis Membrane with Chlorhexidine Using Biopolymer Alginate Dialdehyde as A Facile Green Platform for In Situ Biofouling Control"<br>《Applied Materials & Interfaces》, Vol. 12, No. 33, 23 July 2020 (2020-07-23),<br>ISSN: 1944-8244,<br>    pages 37515-37526, in particular figure 1, sections 2, 4 | 1-31 |
| X | Rashid Khan et al. "Grafting D-amino Acid onto MF Polyamide Nylon Membrane for Biofouling Control Using Biopolymer Alginate Dialdehyde as A Versatile Platform"<br>《Separation and Purification Technology》, Vol. 231, 03 August 2019 (2019-08-03),<br>ISSN: 1383-5866,<br>    pages 1-8, in particular figure 1, sections 2, 4 | 1-31 |
| X | M.Kamran Khan et al. "Alginate Dialdehyde Meets Nylon Membrane: A Versatile Platform for Facile and Green Fabrication of Membrane Adsorbers"<br>*Journal of Materials Chemistry B*, Vol. 6, No. 11, 08 February 2018 (2018-02-08),<br>ISSN: 2050-750X,<br>    pages 1640-1649, in particular figure 1, experimental part | 1-31 |

☑ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **01 November 2021** | **11 November 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**EP 4 190 848 A1**

**INTERNATIONAL SEARCH REPORT**

| International application No. |
|---|
| **PCT/CN2021/116582** |

**C.    DOCUMENTS CONSIDERED TO BE RELEVANT**

| Category* | Citation of document, with indication, where appropriate, of the relevant passages | Relevant to claim No. |
|---|---|---|
| X | M. Kamran Khan et al. "Facile and Green Fabrication of Cation Exchange Membrane Adsorber with Unprecedented Adsorption Capacity for Protein Purification" *Journal of Chromatography A*, Vol. 1521, 15 September 2017 (2017-09-15), ISSN: 0021-9673, pages 19-26, in particular figure 1, section 2 | 1-31 |
| A | CN 111138696 A (UNIVERSITY OF SCIENCE AND TECHNOLOGY BEIJING) 12 May 2020 (2020-05-12) entire document | 1-31 |
| A | CN 105601976 A (SOOCHOW UNIVERSITY) 25 May 2016 (2016-05-25) entire document | 1-31 |

Form PCT/ISA/210 (second sheet) (January 2015)

28

**INTERNATIONAL SEARCH REPORT**
Information on patent family members

International application No.

**PCT/CN2021/116582**

| Patent document cited in search report | | | Publication date (day/month/year) | Patent family member(s) | | | Publication date (day/month/year) |
|---|---|---|---|---|---|---|---|
| CN | 111138696 | A | 12 May 2020 | CN | 111138696 | B | 29 December 2020 |
| CN | 105601976 | A | 25 May 2016 | CN | 105601976 | B | 03 July 2018 |

Form PCT/ISA/210 (patent family annex) (January 2015)

**REFERENCES CITED IN THE DESCRIPTION**

**Patent documents cited in the description**

- CN 202010922336